# EUROPEAN PATENT APPLICATION

(11) **EP 2 316 972 A1**
(43) Date of publication of application: **04.05.2011**
(21) Application number: 09174455.7
(22) Date of filing: 29.10.2009
(51) Int. Cl.: C12Q 1/68

(54) **Circulating miRNAs as non-invasive markers for diagnosis and staging in prostate cancer**

(71) Applicant: Deutsches Krebsforschungszentrum, 69120 Heidelberg (DE); Georg-August Universität Göttingen, 37075 Göttingen (DE); Universitätsklinikum Hamburg-Eppendorf, 20246 Hamburg (DE)
(72) Inventor: Brase, Jan Christoph, 21255 Tostedt (DE); Sültmann, Holger, 67117 Limburgerhof (DE); Kuner, Ruprecht, 73760 Ostfildern (DE); Johannes, Marc, 55596 Waldböckelheim (DE); Beissbarth, Tim, 69221 Dossenheim (DE); Fälth, Maria, 263 93 Höganäs (DE); Schlomm, Thorsten, 20149 Hamburg (DE); Haese, Alexander, 22529 Hamburg (DE); Steuber, Thomas, 22453 Hamburg (DE)
(74) Representative: Dick, Alexander

(57) **Abstract**

The present invention relates to a method for staging prostate cancer in a subject suffering from prostate cancer, said method comprises (a) determining the amount of at least one miRNA selected from the group consisting of miRNA-375, miRNA-141, miRNA-200b, miRNA-516a-3p and miRNA9*, or the amount of a precursor molecule in a sample from said patient and (b) comparing the, thus, determined amount with a reference amount. Moreover, the present invention is concerned with a method for identifying a subject susceptible to prostate cancer therapy and to a method for diagnosing prostate cancer. Further envisaged by the present invention are a kit and a device adapted to carry out the method of the present invention.

SEQUENCE LISTING

<110>  Deutsches Krebsforschungszentrum, Stiftung des öffentlichen
        Rechts

<120>  Circulating miRNAs as non-invasive markers for diagnosis and
       staging in prostate cancer

<130>  DK66722EP

<160>  10

<170>  PatentIn version 3.5

<210>  1
<211>  22
<212>  RNA
<213>  Homo sapiens

<400>  1
uuuguucguu cggcucgcgu ga                                                22

<210>  2
<211>  22
<212>  RNA
<213>  Homo sapiens

<400>  2
uaacacuguc ugguaaagau gg                                                22

<210>  3
<211>  22
<212>  RNA
<213>  Homo sapiens

<400>  3
uaauacugcc ugguaaugau ga                                                22

<210>  4
<211>  18
<212>  RNA
<213>  Homo sapiens

<400>  4
ugcuuccuuu cagagggu                                                     18

<210>  5
<211>  22
<212>  RNA
<213>  Homo sapiens

<400>  5
auaaagcuag auaaccgaaa gu                                                22

<210>  6
<211>  64
<212>  RNA
<213>  Homo sapiens

<400>  6
ccccgcgacg agccccucgc acaaaccgga ccugagcguu uuguucguuc ggcucgcgug      60

aggc                                                                    64

<210>  7
<211>  95
<212>  RNA
<213>  Homo sapiens

<400>  7
cggccggccc uggguccauc uuccaguaca guguuggaug gucuaauugu gaagcuccua      60

acacugucug guaaagaugg cucccgggug gguuc                                  95


<210>  8
<211>  95
<212>  RNA
<213>  Homo sapiens

<400>  8
ccagcucggg cagccguggc caucuuacug ggcagcauug gauggaguca ggucucuaau      60

acugccuggu aaugaugacg gcggagcccu gcacg                                  95


<210>  9
<211>  90
<212>  RNA
<213>  Homo sapiens

<400>  9
ucucaggcug ugaccuucuc gaggaaagaa gcacuuucug uugucugaaa gaaaagaaag      60

ugcuuccuuu cagaggguua cgguuugaga                                        90


<210>  10
<211>  89
<212>  RNA
<213>  Homo sapiens

<400>  10
cgggguuggu uguuaucuuu gguuaucuag cuguaugagu gguguggagu cuucauaaag      60

cuagauaacc gaaaguaaaa auaacccca                                         89

## Description

The present invention relates to a method for staging prostate cancer in a subject suffering from prostate cancer, said method comprises (a) determining the amount of at least one miRNA selected from the group consisting of miRNA-375, miRNA-141, miRNA-200b, miRNA-516a-3p and miRNA9*, or the amount of a precursor molecule in a sample from said patient and (b) comparing the, thus, determined amount with a reference amount. Moreover, the present invention is concerned with a method for identifying a subject susceptible to prostate cancer therapy and to a method for diagnosing prostate cancer. Further envisaged by the present invention are a kit and a device adapted to carry out the method of the present invention.

Prostate cancer is the most frequently diagnosed malignancy and second most cause of cancer related death among western males. As development and progression of cancer is driven by molecular alterations, the analysis of molecular features may eventually allow a better prediction of the behavior of individual cancer.

Prostate cancer has a high incidence but tumor aggressiveness and long time outcome are variable. The separation of high and low risk prostate tumors for the decision on therapy is still an unsolved problem in the clinical setting. The common classification of prostate cancer is based on clinical and pathological parameters, like preoperative prostate-specific antigen (PSA), digital rectal examination (DRE), transrectal ultrasound testing, and the histological grading (Gleason score) of prostate biopsies. However, all these parameters have limitations in their predictive power.

The widespread use of the prostate specific antigen (PSA) for the detection of PCA has resulted in an increasing number of men diagnosed with organ-confined, low Gleason-score PCA, which are potentially curable. However, the high sensitivity of the PSA test is accompanied by a low specificity, causing many patients suffering from unnecessary biopsy taking. Men with normal prostate pathology generally have a PSA level in blood below 4ng/ml. PSA levels between 4ng/ml and 10ng/ml ('grey zone') have a 25% chance of having prostate cancer. As a consequence, in 75% of the cases, men with an abnormal DRE and a PSA in this grey zone have a negative, or a seemingly unnecessary biopsy.

PSA is an indicator of prostate volume and therefore shows limitations in assisting prognosis in prostate cancer. Therefore, a prostate tissue biopsy is taken to confirm the diagnosis and to evaluate tumor aggressivness. The tumor tissue is graded according to its cellular differentiation status using the Gleason Score (GS) system. Currently, GS is the most reliable prognostic marker for clinical risk stratification. However, prostate cancer is a multifocal disease. Thus, often the tumor is missed during biopsy taking, and it is not known whether the tumor focus within a biopsy is the one driving cancer progression. Therefore, a large proportion of tumors have to be upgraded after radical prostatectomy. This upgrading may affect treatment decisions, since tumors with a high GS are associated with postoperative biochemical resurrence and worse survival.

Treatment decisions for an individual patient are linked to the stage of prostate cancer present in that individual. A common classification of the spread of prostate cancer was developed by the American Urological Association (AUA). The AUA system divides prostate tumors into four stages, A to D. Stage A, microscopic cancer within prostate, is further subdivided into stages A1 and A2. Sub-stage A1 is a well-differentiated cancer confined to one site within the prostate. Treatment is generally observation, radical prostatectomy, or radiation. Sub-stage A2 is a moderately to poorly differentiated cancer at multiple sites within the prostate. Treatment is radical prostatectomy or radiation. Stage B, palpable lump within the prostate, is also further subdivided into sub-stages B1 and B2. In sub-stage B1, the cancer forms a small nodule in one lobe of the prostate. In sub-stage B2, the cancer forms large or multiple nodules, or occurs in both lobes of the prostate. Treatment for sub-stages B1 and B2 is either radical prostatectomy or radiation. Stage C is a large cancer mass involving most or all of the prostate and is also further subdivided into two sub-stages. In sub-stage C1, the cancer forms a continuous mass that may have extended beyond the prostate. In sub-stage C2, the cancer forms a continuous mass that invades the surrounding tissue. Treatment for both these sub-stages is radiation with or without drugs to address the cancer. The fourth stage, Stage D is metastatic cancer and is also subdivided into two sub-stages. In sub-stage D1, the cancer appears in the lymph nodes of the pelvis. In sub-stage D2, the cancer involves tissues beyond lymph nodes. Treatment for both of these sub-stages is systemic drugs to address the cancer as well as pain.

However, current prostate cancer staging methods are limited. As many as 50% of prostate cancer initially staged as A2, B, or C are actually stage D, metastatic. Discovery of metastasis is significant because patients with metastatic cancer have a poorer prognosis and require significantly different therapy than those with localized cancer. The five year survival rates for patients with localized and metastatic prostate cancer are 93% and 29%, respectively.

miRNAs are short RNA molecules (19-26 nucleotides), which have been suggested to be important regulators of biological functions (Bushati N, Cohen SM. microRNA functions. Annu Rev Cell Dev Biol 2007;23:175-205). Alterations in miRNA expression can affect important cellular processes like cell cycle, proliferation or apoptosis, thus providing a direct link to cancer development and progression (Calin GA, Croce CM. MicroRNA signatures in human cancer. Nat Rev Cancer 2006;6(11):857-66; Rosenfeld N, Aharonov R, Meiri E, et al. MicroRNAs accurately identify cancer tissue origin. Nat Biotechnol 2008;26(4):462-9).

The possibility to extract and measure stable free miRNA in serum has recently been shown and represents a resource for potential biomarkers. Lawrie et al. were the first to show the presence of miRNAs in body fluids of patients suffering from B-cell lymphoma (Lawrie CH, Gal S, Dunlop HM, et al. Detection of elevated levels of tumour-associated microRNAs in serum of patients with diffuse large B-cell lymphoma. Br J Haematol 2008;141(5):672-5).

Mitchell et al. demonstrated with a mouse model that tumor-derived miRNA enter the circulation even when originated from epithelial cancer types. In this study, they additionally showed that circulating *miRNA-141* was elevated in metastatic prostate cancer when compared to healthy controls (Mitchell PS, Parkin RK, Kroh EM, et al. Circulating microRNAs as stable blood-based markers for cancer detection. Proc Natl Acad Sci U S A 2008;105(30):10513-89).

Circulating miRNAs have also been shown to occur on different levels between tumor patients and healthy controls in various other cancer diseases (Tanaka M, Oikawa K, Takanashi M, et al. Down-regulation of miR-92 in human plasma is a novel marker for acute leukemia patients. PLoS ONE 2009;4(5):e5532; Chen X, Ba Y, Ma L, et al. Characterization of microRNAs in serum: a novel class of biomarkers for diagnosis of cancer and other diseases. Cell Res 2008;18(10):997-1006; Taylor DD, Gercel-Taylor C. MicroRNA signatures of tumor-derived exosomes as diagnostic biomarkers of ovarian cancer. Gynecol Oncol 2008;110(1):13-21). However, cell-free miRNAs have not been found to occur on different levels between different stages in epithelial cancer (Resnick KE, Alder H, Hagan JP, Richardson DL, Croce CM, Cohn DE. The detection of differentially expressed microRNAs from the serum of ovarian cancer patients using a novel real-time PCR platform. Gynecol Oncol 2008; Ng EK, Chong WW, Jin H, et al. Differential expression of microRNAs in plasma of colorectal cancer patients: A potential marker for colorectal cancer screening. Gut 2009).

Thus, a great need exists to identify novel molecular markers that reflect tumor progression to enhance the clinical management of prostate cancer. Particularly, means and methods are required which allow for an easy and reliable staging of prostate cancer as well as for means and methods which allow for a diagnosis of prostate cancer as well as for the identification of subject being susceptible to a therapy that aims to treat prostate cancer. Such reliable means and methods have not been described yet.

The technical problem underlying the present invention can be seen as the provision of means and methods for complying with the aforementioned needs. The technical problem is solved by the embodiments characterized in the claims and herein below.

Accordingly, the present invention relates to a method for staging prostate cancer in a subject suffering from prostate cancer, comprising
a) determining the amount of at least one miRNA selected from the group consisting of miRNA-375, miRNA-141, miRNA-200b, miRNA-516a-3p and miRNA9*, or the amount of a precursor molecule of said at least one miRNA in a sample from said subject,
b) comparing the amount of said at least one miRNA or of said precursor molecule as determined in step a) with a reference amount (with reference amounts), and
c) staging prostate cancer in said subject based on the information obtained in step b).

The method of the present invention, preferably, is an in vitro method. Moreover, it may comprise steps in addition to those explicitly mentioned above. For example, further steps may relate to sample pre-treatments or evaluation of the results obtained by the method. The method of the present invention preferably is used for staging prostate cancer in a subject suffering from prostate cancer. However, the method of the present invention may also be used for monitoring, confirmation, and subclassification of said subject. The method may be carried out manually or assisted by automation. Preferably, step (a) and (b) may in total or in part be assisted by automation, e.g., by a suitable robotic and sensory equipment for the determination in step (a), or a computer-implemented comparison in step (b).

As will be understood by those skilled in the art, the aforementioned staging is usually not intended to be correct for 100% of the subjects to be analyzed. The term, however, requires that the assessment will be valid for a statistically significant portion of the subjects to be analyzed. Whether a portion is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.001. Preferably, the probability envisaged by the present invention allows that the staging will be correct for at least 60%, at least 70%, at least 80%, or at least 90% of the subjects of a given cohort.

The term "subject" as used herein relates to male animals, preferably male mammals, and, more preferably, male humans. However, it is envisaged in accordance with the aforementioned method of the present invention that the subject shall suffer from prostate cancer.

The term "prostate cancer" is known in the art. As used herein, the term, preferably, relates to any proliferative lesion or abnormality of the prostate. Thus, the term, preferably, encompasses pre-malignant lesions, malignant lesions as well as solid tumors and metastatic disease (both locally metastatic and more widely metastatic).

How to assess whether a subject suffers from prostate cancer is well known in the art. The clinically decision making process of men with an elevated risk of PCA is determinated in evidence based clinical guidelines. (Heidenreich A, Aus G, Bolla M, et al. EAU guidelines on prostate cancer. Eur Urol 2008;53:68-80)

The term "staging prostate cancer" as used herein, preferably, means to differentiate between various stages of prostate cancer. The general classification of prostate tumor stages is well-known to the skilled artisan. In brief, the most commonly used staging method is the Tumour, Nodes, Metastasis (TNM) system, which recognises four stages of local tumour growth, from T1 (incidental) to T4 (invasion of neighbouring organs). Each stage describes the state of pathological development of the tumour. T1 represents an 'incidental' state, where the tumour is detected by chance following transurethral resection or by biopsy following PSA testing. At this stage, the tumour will be undetectable by palpation (DRE) or ultrasound, but may be diagnosed by the method of the present invention. T4 represents advanced disease, where the tumour has invaded neighbouring organs. The nodal stage (NO-N1) and the metastatic stage (M0-M1C) reflect the clinical spread of the disease to lymph nodes and distant sites (metastasis), respectively. Grading systems can assess the degree of cell anaplasia (variation in size, shape and staining properties) and differentiation (how well differentiated the cells are) in the tumour. The Gleason grading system is based on the extent to which the tumour cells are arranged into recognisably glandular structures and the level of cell differentiation. The Gleason system identifies more than five levels of increasing disease aggressiveness, with Grade 1 being the least aggressive and over Grade 5 being the most aggressive cancer.

As used herein, the term "staging prostate cancer", preferably, refers to differentiating between a mild and a severe form of prostate cancer. A mild form of prostate cancer in the context of the present invention, preferably, is primary prostate cancer. A severe form of prostate cancer, preferably, is metastasized prostate cancer. Thus, the aforementioned method of the present invention, preferably, allows for differentiating between primary prostate cancer and metastasized prostate cancer.

The term "primary prostate cancer" in the context of the method of the present invention, preferably relates to localized prostate cancer (e.g. pT2 or less).

The term "metastasized prostate cancer" in the context of the method of the present invention, preferably, relates to prostate cancer with metastasized tumors, preferably bone-metastasis tumors (e.g.prostate cancer pN+, M+, systemic stage).

In a preferred embodiment of the method of the present invention, it is differentiated between high risk, an intermediate risk and a low risk.

A subject being at "low risk" in the context of the method of the present invention relates to subject suffering from prostate cancer with a Gleason score of 6 or lower than 6.

A subject being at "intermediate risk" in the context of the method of the present invention relates to subject suffering from prostate cancer with a Gleason score of 7.

A subject being at "high risk" in the context of the method of the present invention relates to subject suffering from prostate cancer with a Gleason score of 8 or larger than 8.

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Preferred tissue and organ sample are obtained from the prostate which may be obtained by well-known methods, preferably by needle biopsy. Separated cells may be obtained from the body fluids or the tissues or organs by separating techniques such as centrifugation or cell sorting. Preferred cells in the context of the method of the present invention are prostate cells. The most preferred sample is the context of the present invention is a blood, blood serum, or a blood plasma sample.

In a preferred embodiment of the method of the present invention, the sample is further processed by well known methods in order to further enrich and/or purify the miRNA molecules or precursor molecules thereof as referred to herein. The further processing may depend on the nature of the marker, i.e. whether the marker to be determined is a miRNA or a precursor molecule. Preferably, if the marker is a miRNA, then small RNAs (e.g. 18 to 24 nt in length) are enriched and/or purified by methods well known by the skilled person (after obtaining the sample). Preferably, if the biomarker is a precursor molecule, said total RNA may be enriched and/or purified by methods well known in the art (after obtaining the sample).

For enriching miRNA molecules from a sample, total RNA may be purified from the sample in a first step. In a second step the total RNA may be separated by polyacrylamide gel electrophoresis. In the third step, the fraction of small RNAs (e.g. 18 to 24 nucleotides in length) may be purified from the polyacrylamide gel. In the context of the present invention it is also contemplated to isolate miRNAs from the sample. Particularly, a combination of phenol/guanidine-based lysis and silicamembrane-based purification may be used for the isolation of cell-free RNA from serum samples.

Moreover, various miRNA mimics (e.g. from *c*. *elegans cel-miRNA-39, cel-miRNA-54, cel-miRNA-238,*see Examples) may be added to the sample as a spike-in control for purification efficiency (Mitchell PS, Parkin RK, Kroh EM, et al. Circulating microRNAs as stable blood-based markers for cancer detection. Proc Natl Acad Sci U S A 2008;105(30):10513-89)

It is further contemplated that a reverse transcription is carried out after obtaining the RNA sample (particularly in order to quantify the RNA by quantitative RT-PCR, see herein below).

microRNAs (miRNA) are single-stranded RNA molecules of app. 21-23 nucleotides in length which regulate gene expression. Said miRNAs are frequently also referred to as mature miRNAs. miRNAs are non-coding RNAs and, thus, are not translated into protein. They are derived from a primary transcript (a pri-miRNA) which is processed in a first step into a short stem-loop (hairpin) structure (pre-miRNA) and then into a functional miRNA. Mature miRNA molecules are partially complementary to corresponding target mRNAs and binding leads to translation inhibition or mRNA degradation.

In the context of the present invention, the amount of at least one miRNA selected from the group consisting of miRNA-375, miRNA-141, miRNA-200b, miRNA-516a-3p and miRNA9*, or of a precursor molecule thereof shall be determined in a sample from a subject.

The aforementioned miRNAs are single stranded RNA molecules. The sequence of miRNA-375 is shown in SEQ ID NO:1, the sequence of miRNA-141 is shown in SEQ ID NO:2, the sequence of miRNA-200b is shown in SEQ ID NO:3, the sequence of miRNA-516a-3p is shown in SEQ ID NO:4, and the miRNA9* is shown in SEQ ID NO:5.

The phrase "at least one" as used herein, preferably, means "one miRNA", or "more than one", particularly, two, three, four or five miRNAs or precursors thereof Thus, it is contemplated to stage prostate cancer by determining the amounts of various miRNAs or precursors thereof.

Precursor molecules of the aforementioned miRNAs are, preferably, the corresponding pri-miRNAs and, more preferably, the corresponding pre-miRNAs. The sequence of the pre-miRNA precursor of miRNA-375 is shown in SEQ ID NO:6, the sequence of the pre-miRNA precursor of miRNA-141 is shown in SEQ ID NO:7, the sequence of the pre-miRNA precursor of miRNA-200b is shown in SEQ ID NO:8, and the sequence of the pre-miRNA precursor of miRNA-516a-3p is shown in SEQ ID NO:9, and the sequence of the pre-miRNA precursor of miRNA9* is shown in SEQ ID NO:10.

It is also contemplated that the amount of a variant of any of the aforementioned SEQ ID Nos may be determined in a sample of a subject. Such variants, preferably, have a nucleic acid sequence being at least 70%, at least 80%, at least 90%, at least 95%, at least 96%, at least 97%, at least 98% or at least 99% identical to any one of SEQ ID NOs:1 to 10. The degree of identity (percentage, %) between two or more nucleic acid sequences is, preferably, determined by the algorithms of Needleman and Wunsch or Smith and Waterman. To carry out the sequence alignments, the program PileUp (J. Mol. Evolution., 25, 351-360, 1987, Higgins 1989, CABIOS, 5: 151-153) or the programs Gap and BestFit (Needleman 1970, J. Mol. Biol. 48; 443-453 and Smith 1981, Adv. Appl. Math. 2; 482-489), which are part of the GCG software packet (Genetics Computer Group, 575 Science Drive, Madison, Wisconsin, USA 53711, vers. 1991), are to be used. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

It is to be understood the diagnosis/differentiation in the context of the present invention may also be carried out by determining the amount of a part of the pre-miRNA. For example, the diagnosis may be carried out by determining, in a sample from a subject, the amount of the loop of the miRNA-precursor molecule of the corresponding miRNA* molecule, i.e. of the small RNA processed from the hairpin arm opposite to the mature miRNA.

Determining the amount of a miRNA molecule or of a precursor molecule thereof can be can be done by any method deemed appropriate. A preferred method for determining said amounts is described in the Examples. A preferred method is also described by Mitchell et al., which hereby is incorporated by reference with respect to its entire disclosure content (Mitchell PS, Parkin RK, Kroh EM, et al. Circulating microRNAs as stable blood-based markers for cancer detection. Proc Natl Acad Sci U S A 2008;105(30):10513-89).

The amount of a miRNA or of a precursor molecule thereof may be determined by using a probe oligonucleotide that specifically detects the miRNA or of a precursor molecule to be analyzed or of an amplification product of said miRNA or said precursor (For an explanation of the term "amplification products", see below).

The determination of the amount of a miRNA or of a precursor molecule thereof, by specific probe oligonucleotides, preferably, comprises the step of hybridizing a miRNA or of a precursor molecule thereof or of an amplification product (thereof with a probe oligonucleotide that specifically binds to the transcript or the amplification product thereof. A probe oligonucleotide in the context of the present invention, preferably, is a single-stranded nucleic acid molecule that is specific for said miRNA or of a precursor molecule thereof and, preferably, comprises a stretch of nucleotides that specifically hybridizes with the target and, thus, is complementary to the target polynucleotide. Said stretch of nucleotides is, preferably, 85%, 90%, 95%, 99% or more preferably 100% identical to a sequence region comprised by a target polynucleotide. If the target molecule is a miRNA the probe oligonucleotide is, preferably, 85%, 90%, 95%, 99% or more preferably 100% identical to said miRNA. The degree of identity (percentage, %) between two or more nucleic acid sequences is, preferably, determined by the algorithms of Needleman and Wunsch or Smith and Waterman. To carry out the sequence alignments, the program PileUp (J. Mol. Evolution., 25, 351-360, 1987, Higgins 1989, CABIOS, 5: 151-153) or the programs Gap and BestFit (Needleman 1970, J. Mol. Biol. 48; 443-453 and Smith 1981, Adv. Appl. Math. 2; 482-489), which are part of the GCG software packet (Genetics Computer Group, 575 Science Drive, Madison, Wisconsin, USA 53711, vers. 1991), are to be used. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

The probe oligonucleotide may be labelled or contain other modifications including enzymes which allow a determination of the amount of a miRNA or precursor molecule thereof (or of an amplification product thereof). Labelling can be done by various techniques well known in the art and depending of the label to be used. Preferred labels are described elsewhere in this specification.

In a preferred embodiment of the method of the present invention, the determination of the amount of the miRNA (or of the precursor molecule thereof) comprises the step of amplifying the miRNA molecule (or of the precursor molecule thereof) comprised by the sample and determining the amount of the, thus, generated amplification products. By determining the amounts of said amplification products, the amount of the miRNA molecules (or of the precursor molecules thereof) can be assessed. Preferably, the amounts of the amplifications products are determined by using probe oligonucleotides as described herein above.

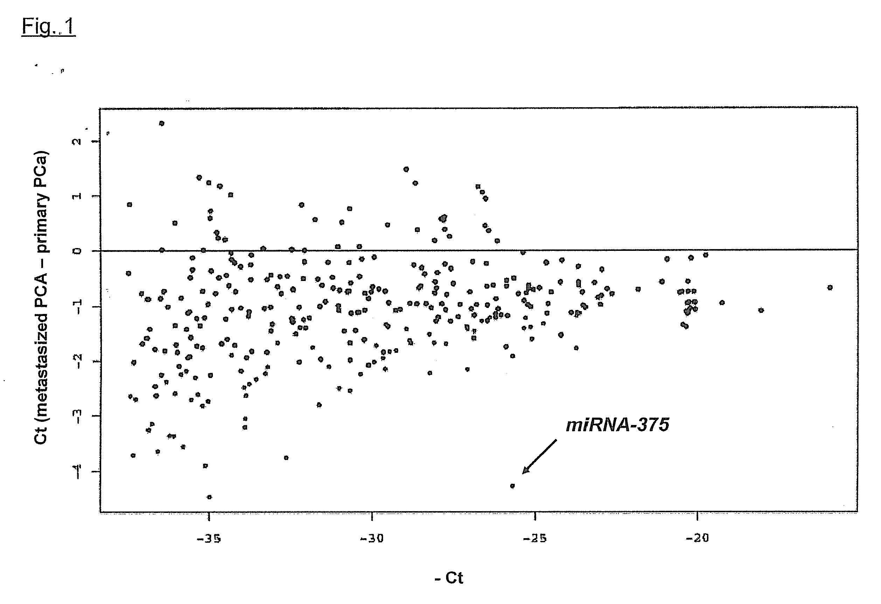

If the amount of an amplification product of a miRNA shall be determined in a sample, the miRNA may be amplified as described by Mitchell et al. (loc. cit, see e.g. Fig 1a of the document Mitchell). The miRNAs are ligated at the 3' and the 5' end to single-stranded oligonucleotides that contain universal sequences for reverse transcription and for PCR. A reverse transcription of the ligation product is carried out. After reverse transcription, the resulting products are amplified by PCR.

In a more preferred embodiment of the method of the present invention, said PCR is a real-time PCR (RT-PCR). Thus, the amount of a miRNA is, preferably, determined by quantitative real-time PCR.

The term "real-time PCR" is known in the art. When real-time PCR is carried out, a signal emitted from the PCR assay is monitored during the PCR reaction as an indicator of the amount of amplification product during each PCR amplification cycle, (as opposed to conventional PCR methods, in which is the amplification product is detected at the endpoint of the PCR reaction. Real-time PCR is generally based on the detection and quantification of a fluorescent reporter. The signal increases in proportion to the amount of PCR product in a reaction.

When carrying out real-time PCR, the amount of a miRNA or of a precursor molecule thereof may be determined by comparing the results to a standard curve produced by real-time PCR of serial dilutions (e.g. undiluted, 1:4, 1:16, 1:64, 1:128) of a pre-determined amount of said marker. Moreover, to accurately determine the amount, the measured amount may be divided by the amount of normalization RNA (or DNA) which allows normalizing for possible variation of the amount of RNA between different samples. A normalization RNA can be, preferably, RNA (or DNA) which is added to the sample (spike-in, see Examples).

Moreover, it is contemplated to determine the amount of a marker of the present invention by in situ hybridization, preferably, in a prostate tissue sample.

The term "amount" as used herein encompasses the absolute amount of a miRNA or a precursor molecule (or an amplification product thereof), the relative amount or concentration thereof as well as any value or parameter which correlates thereto. Such values or parameters comprise intensity signal values from all specific physical or chemical properties obtained therefrom by direct measurements, e.g., intensity values. Moreover, encompassed are all values or parameters which are obtained by indirect measurements specified elsewhere in this description, e.g., expression levels determined from biological read out systems. It is to be understood that values correlating to the aforementioned amounts or parameters can also be obtained by all standard mathematical operations. If Real-time PCR is carried out, it is particularly preferred to determine the Ct-value (Ct: Cycle threshold) which indicates the relative amount of a marker to be analyzed in a sample. How to determine a Ct-value is well known in the art. In general, the larger the amount of a marker in a sample, the lower the Ct-value.

The term "comparing" as used herein encompasses comparing the amount the miRNA, the precursor molecule thereof comprised by the sample to be analyzed (or of an amplification product of said miRNA or precursor molecule) with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format. Based on the comparison of the amount determined in step a) and the reference amount, it is possible to stage prostate cancer in a sample from a subject suffering from prostate cancer.

The term "reference amount" refers to an amount which allows for staging prostate cancer in a subject suffering from prostate cancer. A reference amount (reference amounts) may be derived from samples of subjects suffering from prostate cancer in various stages.

For example, if it shall be differentiated between primary prostate cancer and metastasized prostate cancer, the reference amounts may be derived from a sample of (i) a subject suffering from primary prostate cancer and/or (ii) a subject suffering from metastasized prostate cancer.

For example, if it shall be differentiated between a low risk, a intermediate risk and a high risk, the reference amounts may be derived from a sample of (i) a subject being at low risk, (ii) a subject being at intermediate risk, and/or (iii) a subject being at high risk.

The reference can also be the average or mean obtained from a group of such samples. Preferably, said references are from blood, blood plasma or blood serum samples. If the reference shall be obtained from a biopsy tissue sample, the said sample shall comprise or essentially consists of prostate cancer tissue.

The reference results may be obtained by applying the method of the present invention. The reference can also be the average or mean obtained from a group of the aforementioned samples. Moreover, the reference, also preferably, could be a calculated reference, most preferably the average or median, for the relative or absolute amount of a marker as set forth in the context of the method of the present invention of a representative population of individuals which are in various stages (preferably the aforementioned stages) with respect to prostate cancer, preferably, the US, Asian or European population The absolute or relative amounts of said markers of said individuals of the population can be determined as specified elsewhere herein. How to calculate a suitable reference value, preferably, the average or median, is well known in the art. The population of subjects referred to before shall comprise a plurality of subjects, preferably, at least 5, 10, 50, 100, 1,000 or 10,000 subjects. It is to be understood that the subject to be diagnosed by the method of the present invention and the subjects of the said plurality of subjects are of the same species.

More preferably, a "reference" will be obtained by determining the values for the at least one characteristic feature for a group of reference subjects, e.g. a group of subjects suffering from primary prostate cancer, a group of subjects suffering from metastasized prostate cancer, a population comprising the subject to be investigated; and calculating the reference by appropriate statistic measures including those referred to elsewhere herein, such as median, average, quantiles, PLS-DA, logistic regression methods, random forest classification or others that give a threshold value. The threshold value should take the desired clinical settings of sensitivity and specificity of the diagnostic and prognostic test into consideration.

The reference amount applicable for an individual subject may vary depending on various physiological parameters such as age, or subpopulation, as well as on the means used for the determination of a marker referred to herein. A suitable reference amount may be determined by the method of the present invention from a reference sample to be analyzed together, i.e. simultaneously or subsequently, with the test sample.

Reference amounts of the diagnostic markers referred to herein can be established, and the amount of the marker in a sample from a subject can simply be compared to the reference amount. The sensitivity and specificity of a diagnostic and/or prognostic test depends on more than just the analytical "quality" of the test-they also depend on the definition of what constitutes an abnormal result. In practice, Receiver Operating Characteristic curves, or "ROC" curves, are typically calculated by plotting the value of a variable versus its relative frequency in "normal" and "disease" populations. For any particular marker, a distribution of marker levels for subjects in various stages of prostate cancer may overlap. Under such conditions, a test may not absolutely distinguish with 100% accuracy, and the area of overlap indicates where the test cannot distinguish normal from disease. A threshold is selected, above which (or below which, depending on how a marker changes with the disease) the test is considered to be abnormal and below which the test is considered to be normal. The area under the ROC curve is a measure of the probability that the perceived measurement will allow correct identification of a condition. ROC curves can be used even when test results do not necessarily give an accurate number. As long as one can rank results, one can create an ROC curve.

Preferably, if it shall be differentiated between primary prostate cancer and metastasized prostate cancer, and if the reference results are obtained from (i) a subject suffering from primary prostate cancer and (ii) as subject suffering from metastasized prostate cancer, the staging of prostate cancer can be carried out on the degree of identity or similarity between the test results obtained from the test sample and the aforementioned reference results, i.e. based on an identical or similar qualitative or quantitative composition with respect to the at least one miRNA marker or precursor molecule thereof. The results of the test sample and the reference results are identical, if the values for the characteristic features and, in the case of quantitative determination, the intensity values are identical. Said results are similar, if the values of the characteristic features are identical but the intensity values are different. Such a difference is, preferably, not significant and shall be characterized in that the values for the intensity are within at least the interval between 1^{st} and 99^{th} percentile, 5^{th} and 95^{th} percentile, 10^{th} and 90^{th} percentile, 20^{th} and 80^{th} percentile, 30^{th} and 70^{th} percentile, 40^{th} and 60^{th} percentile of the reference value, the 50^{th}, 60^{th}, 70^{th}, 80^{th}, 90^{th} or 95^{th} percentile of the reference value.

Preferably, the same applies if it shall be differentiated between a low risk, a intermediate risk and a high risk and if the reference results are obtained from (i) a subject being at low risk, (ii) a subject being at intermediate risk and (iii) as subject being at high risk.

It is also contemplated in the context of the method of the present invention, that the staging may be based differences between the test results obtained from the test sample and the aforementioned reference results, i.e. differences in the quantitative composition with respect to the at least one marker. The same applies if a calculated reference as specified above is used. The difference, preferably, shall be an increase in the absolute or relative amount of a diagnostic marker according to present invention. Preferably, the increase in the relative or absolute amount is significant, i.e. outside of the interval between 45^{th} and 55^{th} percentile, 40^{th} and 60^{th} percentile, 30^{th} and 70^{th} percentile, 20^{th} and 80^{th} percentile, 10^{th} and 90^{th} percentile, 5^{th} and 95^{th} percentile, 1^{st} and 99^{th} percentile of the reference value.

Moreover, it is also contemplated that a threshold amount can be used as a reference amount. An amount of a marker as referred to herein in test sample which is above the threshold amount will be indicative for a severe from of prostate cancer, wherein an amount below the threshold amount will be indicative for a mild form of prostate cancer.

Advantageously, the studies carried out in the context of the present invention have shown that determining the amount of at least one miRNA selected from the group consisting of miRNA-375, miRNA-141, miRNA-200b, miRNA-516a-3p and miRNA9*, or the amount of a precursor molecule of said at least one miRNA in a plasma sample from said subject, and comparing the, thus, determined amount to reference amounts allows for reliably staging prostate cancer in a subject suffering from prostate cancer. Particularly, a screening study was performed in order to identify circulating miRNAs in the serum correlated with prostate tumor progression by monitoring 667 human miRNAs in two clinical risk groups. miRNA-375, miRNA-9*, miRNA-141, miRNA-200b and miRNA-516a-3p were identified to be highly abundant in serum from patients with a metastasized prostate cancer when compared to primary prostate cancer.

Moreover, the identified miRNAs (miRNA-375, miRNA-9*, miRNA-141, miRNA-200b and miRNA-516a-3p) were analyzed in a prospective validation study using serum samples of an independent patient cohort (see Examples). The validation study proved that the selected circulating miRNAs are considerably correlated to tumor progression. Thus, it has been shown circulating miRNAs correlate with clinico-pathological endpoints in prostate cancer.

The identified miRNAs correlated with tumor progression, in particular, miRNA-375 which correlated to Gleason score, lymph-node status and pT stage. Circulating miRNA-375 were compared with preoperative PSA values in the studies underlying the present invention. It was shown that miRNA-375 exhibited considerably higher levels in the sera of patients with high risk tumors (Gleason Score >=8 or patients with a metastasized prostate cancer) compared to lower risk tumors. In contrast, no significant differences were detected for the comparison of the PSA levels between Gleason 7 and Gleason >=8 tumors as well as lymph-node status. Thus, miRNA-375 allows a more reliable staging of prostate cancer than PSA.

miRNA-375 revealed a significant difference in three groupwise comparisons between low, intermediate and high risk. Additionally, miRNA-375 was significantly higher abundant in the sera of patients with lymph-node positive status or pathological stage pT3. In order to assess the influence of PSA and miRNA-375 on the clinical prognosis of pathological endpoints, an ANOVA analysis was employed (see Examples). Overall, the ANOVA analysis indicated that miRNA-375 is an additional non-invasive marker for prostate cancer, which - if applied- will contribute to the amelioration of therapeutic decisions in the clinical setting.

The method of the present invention is particularly advantageous since it can be carried out with small volumes of a blood, blood serum or blood plasma sample. Therefore, biopsies can be avoided.

The explanations and definitions given herein above apply *mutatis mutandis* to the following methods (except stated otherwise).

Moreover, the present invention relates to a method for diagnosing prostate cancer in a subject suspected to suffer from prostate cancer, said method comprising
a) determining the amount of at least one miRNA selected from the group consisting of miRNA-375, miRNA-141, miRNA-200b, miRNA-516a-3p and miRNA9*, or the amount of a precursor molecule of said at least one miRNA in a blood, blood serum or blood plasma sample from said subject,
b) comparing the amount of said at least one miRNA or of said precursor molecule as determined in step a) with a reference amount (with reference amounts), and
c) diagnosing prostate cancer in said subject based on the information obtained in step b).

The subject in the context with the aforementioned method shall be suspected to suffer from prostate cancer. Moreover, it is also contemplated that the subject may have a predisposition for prostate cancer.

A subject suspected to suffer from prostate cancer or to have a predisposition therefor as used herein refers to a subject which shows, preferably, symptoms or clinical signs or parameters indicative for prostate cancer. However, the term also relates to an apparently healthy subject, i.e. a subject not exhibiting any of the aforementioned symptoms, clinical signs or parameters. Apparently healthy subjects may by investigated by the method of the present invention as a measure of preventive care or for population screening purposes.

The term "reference amount" as used in the context with the aforementioned method refers to an amount which allows diagnosing prostate cancer in subject suspected to suffer from prostate cancer. A reference amount may either be derived from (i) a subject who suffers from prostate cancer or (ii) a sample from a subject known not to suffer from prostate cancer.

The reference can also be the average or mean obtained from a group of such samples. The reference results may be obtained by applying the method of the present invention. Alternatively, but nevertheless also preferred, the reference results may be obtained from sample from a subject known not to suffer from prostate cancer or a subject known not to have a predisposition therefore, i.e. a healthy subject with respect to prostate cancer and, more preferably, other diseases, in particular cancer diseases, as well. Likewise, if the reference shall be obtained from a biopsy tissue sample, the said sample shall essentially consist of apparently healthy prostate tissue. The reference can also be the average or mean obtained from a group of such samples. Preferably, if biopsy tissues samples are envisaged, the sample underlying the reference and the test sample can be obtained from the same subject, i.e. from areas which are apparently affected by prostate cancer and from areas suspected to be affected by prostate cancer. Moreover, the reference, also preferably, could be a calculated reference, most preferably the average or median, for the relative or absolute amount of a marker as referred to herein of a representative population of individuals which are apparently healthy or suffer from prostate cancer, wherein the subjects suffering from prostate cancer are within the prevalence for the disease in a given population, preferably, the US, Asian or European population The absolute or relative amounts of said markers of said individuals of the population can be determined as specified elsewhere herein. How to calculate a suitable reference value, preferably, the average or median, is well known in the art. The population of subjects referred to before shall comprise a plurality of subjects, preferably, at least 5, 10, 50, 100, 1,000 or 10,000 subjects. It is to be understood that the subject to be diagnosed by the method of the present invention and the subjects of the said plurality of subjects are of the same species.

More preferably, a "reference" will be obtained by determining the values for the at least one characteristic feature for a group of reference subjects, i.e. a group of subjects known to suffer from prostate cancer, a group of subjects known not to suffer from prostate cancer, a population comprising the subject to be investigated or a group of tissue biopsy samples of prostate cancer tissue or apparently healthy tissue and calculating the reference by appropriate statistic measures including those referred to elsewhere herein.

The reference amount applicable for an individual subject may vary depending on various physiological parameters such as age, or subpopulation, as well as on the means used for the determination of a marker referred to herein. A suitable reference amount may be determined by the method of the present invention from a reference sample to be analyzed together, i.e. simultaneously or subsequently, with the test sample.

Reference amounts of the diagnostic markers referred to herein can be established, and the amount of the marker in a sample from a subject can simply be compared to the reference amount. The sensitivity and specificity of a diagnostic and/or prognostic test depends on more than just the analytical "quality" of the test-they also depend on the definition of what constitutes an abnormal result. In practice, Receiver Operating Characteristic curves, or "ROC" curves, are typically calculated by plotting the value of a variable versus its relative frequency in "normal" and "disease" populations (see also above).

In case a the reference results are obtained from a subject or a group known to suffer from prostate cancer, the said disease can be diagnosed based on the degree of identity or similarity between the test results obtained from the test sample and the aforementioned reference results, i.e. based on an identical or similar qualitative or quantitative composition with respect to the at least one marker referred to herein. The results of the test sample and the reference results are identical, if the values for the characteristic features and, in the case of quantitative determination, the intensity values are identical. Said results are similar, if the values of the characteristic features are identical but the intensity values are different. Such a difference is, preferably, not significant and shall be characterized in that the values for the intensity are within at least the interval between 1^{st} and 99^{th} percentile, 5^{th} and 95^{th} percentile, 10^{th} and 90^{th} percentile, 20^{th} and 80^{th} percentile, 30' and 70^{th} percentile, 40^{th} and 60^{th} percentile of the reference value.the 50^{th}, 60^{th}, 70^{th}, 80^{th}, 90^{th} or 95^{th} percentile of the reference value.

In case the reference results are obtained from a subject or a group known not to suffer from prostate cancer, the said disease can be diagnosed based on the differences between the test results obtained from the test sample and the aforementioned reference results, i.e. differences in the qualitative or quantitative composition with respect to the at least one marker referred to herein. The same applies if a calculated reference as specified above is used. The difference, preferably, shall be an increase in the absolute or relative amount of a diagnostic marker according to present invention. Preferably, the increase in the relative or absolute amount is significant, i.e. outside of the interval between 45^{th} and 55^{th} percentile, 40^{th} and 60^{th} percentile, 30^{th} and 70^{th} percentile, 20^{th} and 80^{th} percentile, 10^{th} and 90^{th} percentile, 5^{th} and 95^{th} percentile, 1^{st} and 99^{th} percentile of the reference value.

Thus, the reference may be derived from a subject known to suffer from a prostate cancer. Preferably, identical or similar results for the test sample and the reference are indicative for a subject suffering from prostate carcinoma.

As set forth above, the reference may be also derived from a subject known to not suffer from a prostate cancer. An amount of a diagnostic marker according to the present invention larger than the aforementioned reference, preferably, indicates that the subject suffers from prostate cancer.

Moreover, it is also contemplated that a threshold amount can be used as a reference amount. An amount of a marker as referred to herein in test sample which is above the threshold amount will be indicative for a subject suffering from prostate cancer, wherein an amount below the threshold amount will be indicative for a subject not suffering from prostate cancer.

Advantageously, it has been shown that the markers as referred to herein are increased in samples from subjects suffering from prostate cancer as compared to samples from subjects not suffering from prostate cancer. Thus, the determination of said markers allows for a reliable diagnosis of prostate cancer in a subject suspected to suffer from prostate cancer. The aforementioned method is particularly advantageous, if the amount of said marker is determined in a blood, blood plasma or blood serum sample, since these samples can be easily obtained.

As set forth elsewhere herein already, the classification and the determination of progression is important in order to allow for efficient and appropriate therapeutic interventions. In particular, it is desirable to start therapeutic interventions prior to progression of the prostate cancer into stages where metastasis occurs, such as the stages 3a and 3b.

The determination of various miRNAs (or of the precursor molecules thereof) allows for reliably staging prostate cancer (see herein above). Since the treatment of prostate cancer depends on the stage of prostate cancer, the determination of these markers, allows for making decisions regarding the treatment of a patient suffering from prostate cancer.

Accordingly, the present invention relates to a method for identifying a subject susceptible to prostate cancer therapy, said method comprising
a) determining the amount of at least one miRNA selected from the group consisting of miRNA-375, miRNA-141, miRNA-200b, miRNA-516a-3p and miRNA9*, or the amount of a precursor molecule of said at least one miRNA in a sample from a subject suffering from prostate cancer,
b) comparing the amount of said at least one miRNA or of said precursor molecule as determined in step a) with a reference amount (with reference amounts), and
c) identifying a subject susceptible to prostate cancer therapy.

The definitions and explanations made herein apply mutatis mutandis to the aforementioned method (except stated otherwise).

The aforementioned method allows to identify a subject being susceptible to prostate cancer therapy or not, and, thus, to determine whether a subject who suffers from prostate cancer will benefit from prostate cancer therapy or not. Suitable prostate cancer therapies include surgery, irradiation, and, more preferably, systemic chemotherapy. Said therapies are well known in the art and, e.g., described in Heidenreich A, Aus G, Bolla M, et al. EAU guidelines on prostate cancer. Eur Urol 2008;53:68-80 or in NCCN Clinical Practice Guidelines in Oncology™ Prostate Cancer, Version 1.2008.

It will be understood that a reference to be, preferably, used for the aforementioned method identifying a subject been susceptible to prostate cancer therapy shall be derived from a subject known to suffer from a subject suffering from a locally advanced prostate cancer (pT2) or a systemic stage prostate cancer (pN+, M+). More preferably, identical or similar results for the test sample and the reference are indicative for a subject been susceptible to prostate cancer therapy in such a case. Alternatively, the reference may be, preferably, derived from a subject known to not to suffer from a locally advanced prostate cancer (pT2) or a systemic stage prostate cancer (pN+, M+) and, thus, e.g. from a subject suffering from localized prostate cancer (pT2). More preferably, an amount of a miRNA or of a precursor thereof which is increased (preferably statistically significant increased) as compared to said reference is indicative for a subject being susceptible to prostate cancer therapy. Whether an increase is statistically significant can be determined by the person skilled in the art without further ado.

The present invention further relates to a method for diagnosing the progression of prostate cancer comprising:
(a) determining the amount of least one miRNA selected from the group consisting of miRNA-375, miRNA-141, miRNA-200b, miRNA-516a-3p and miRNA9*, or the amount of a precursor molecule of said at least one miRNA in a first sample from a subject suffering from prostate carcinoma, and
(b) determining the amount of least one miRNA or of said precursor molecule of said at least one miRNA in a second sample of said subject, and
(c) comparing the amount in said first sample with said amount in said second sample.

Preferably, an increase, and, more preferably, a statistically significant increase of the amount of the marker in the second sample as compared to the amount in the first sample indicates progressing prostate cancer.

The terms "progression of prostate cancer" and "progressing prostate cancer" relate to the conversion of prostate carcinomas from one stage to another, preferably, to a more advanced stage. Preferably, the progression of prostate carcinomas envisaged by the method of the present invention is progression from a localized (pT2) to a locally advanced (pT3) or systemic stage (pN+, M+). As set forth elsewhere herein already, the classification and the determination of progression is important in order to allow for efficient and appropriate therapeutic interventions.

Preferably, the second sample is obtained within four to six months, within six to twelve months, within twelve to 24 months, and, more preferably, within 24 to 48 months after said first sample.

As set forth above, an increase of the amount of the marker in said second sample as compared to the amount of said marker in said first sample indicates progression of prostate cancer. Preferably, said increase is statistically significant. Whether an increase is statistically significant can be determined by the skilled person without further ado. Preferred increases indicating prostate cancer progression are 20%, 30%, 40%, 50% and more preferably, 100 %.

The present invention, furthermore, contemplates a method for monitoring the success of a prostate carcinoma therapy or a therapy against progressing prostate carcinoma. The method shall again comprise the steps of the aforementioned methods of the present invention and identifying a successful therapy based on the diagnostic result. It will be understood that a successful therapy shall result in a decrease (preferably, a significant decreased) of the at least one biomarker from a diseased or advanced into a healthy or less advanced disease stage.

Moreover, the present invention relates to kits and devices adapted to carry out the method of the present invention.

Accordingly, the present invention relates to a device adapted for staging prostate cancer in a subject suffering from prostate cancer comprising:
a) an analysing unit comprising a detection agent for at least one miRNA selected from the group consisting of miRNA-375, miRNA-141, miRNA-200b, miRNA-516a-3p and miRNA9*, or of a precursor molecule thereof, wherein said analyzing unit is adapted for determining the amount(s) of at said at least one miRNA molecule or of a precursor molecule thereof in a sample detected by the detection agent, and
b) an evaluation unit comprising a computer comprising tangibly embedded a computer program code for carrying out a comparison of the determined amount(s) obtained from the analyzing unit with a reference amount (reference amounts).

Moreover, the present invention relates to a device adapted for diagnosing prostate cancer in a subject suspected to suffer from prostate cancer comprising:
a) an analysing unit comprising a detection agent for at least one miRNA selected from the group consisting of miRNA-375, miRNA-141, miRNA-200b, miRNA-516a-3p and miRNA9*, or of a precursor molecule thereof, wherein said analyzing unit is adapted for determining the amount(s) of at said at least one miRNA molecule or of a precursor molecule thereof in a sample detected by the detection agent, and
b) an evaluation unit comprising a computer comprising tangibly embedded a computer program code for carrying out a comparison of the determined amount(s) obtained from the analyzing unit with a reference amount (reference amounts).

Moreover, the present invention relates to a device adapted for identifying a subject being susceptible to prostate cancer therapy comprising:
a) an analysing unit comprising a detection agent for at least one miRNA selected from the group consisting of miRNA-375, miRNA-141, miRNA-200b, miRNA-516a-3p and miRNA9*, or of a precursor molecule thereof, wherein said analyzing unit is adapted for determining the amount(s) of at said at least one miRNA molecule or of a precursor molecule thereof in a sample detected by the detection agent, and
b) an evaluation unit comprising a computer comprising tangibly embedded a computer program code for carrying out a comparison of the determined amount(s) obtained from the analyzing unit with a reference amount (reference amounts).

The term "device" as used herein relates to a system of means comprising at least the aforementioned means operatively linked to each other as to allow the prediction. Preferred means for determining the amount of the markers referred to herein and means for carrying out the comparison are disclosed above in connection with the method of the invention. How to link the means in an operating manner will depend on the type of means included into the device. For example, where means for automatically determining the amount of the said markers are applied, the data obtained by said automatically operating means can be processed by, e.g., a computer program in order to diagnose or distinguish between the diseases referred to herein. Preferably, the means are comprised by a single device in such a case. Said device may accordingly include an analyzing unit for the measurement of the amount of the markers in a sample and a computer unit for processing the resulting data for the differential diagnosis. Preferred means for detection are disclosed in connection with embodiments relating to the method of the invention above. In such a case, the means are operatively linked in that the user of the system brings together the result of the determination of the amount and the diagnostic value thereof due to the instructions and interpretations given in a manual. The means may appear as separate devices in such an embodiment and are, preferably, packaged together as a kit. The person skilled in the art will realize how to link the means without further ado. Preferred devices are those which can be applied without the particular knowledge of a specialized clinician, e.g., electronic devices which merely require loading with a sample. The results may be given as output of parametric diagnostic raw data, preferably, as absolute or relative amounts. It is to be understood that these data will need interpretation by the clinician. However, also envisage are expert system devices wherein the output comprises processed diagnostic raw data the interpretation of which does not require a specialized clinician. Further preferred devices comprise the analyzing units/devices or evaluation units/devices referred to above in accordance with the method of the invention.

Moreover, the present invention relates to a kit adapted for staging prostate cancer in a subject suffering from prostate cancer comprising comprising:
a) a detection agent for at least one miRNA selected from the group consisting of miRNA-375, miRNA-141, miRNA-200b, miRNA-516a-3p and miRNA9*, or of a precursor molecule thereof, and
b) a device adapted for determining the amount of said least one miRNA molecule or of a precursor molecule thereof detected by said detection agent, wherein said device is also adapted for carrying out the comparison of said amount(s) with a reference amount (reference amounts).

Moreover, the present invention relates to a kit adapted for diagnosing prostate cancer in a subject suspected to suffer from prostate cancer comprising:
a) a detection agent for at least one miRNA selected from the group consisting of miRNA-375, miRNA-141, miRNA-200b, miRNA-516a-3p and miRNA9*, or of a precursor molecule thereof, and
b) a device adapted for determining the amount of said least one miRNA molecule or of a precursor molecule thereof detected by said detection agent, wherein said device is also adapted for carrying out the comparison of said amount(s) with a reference amount (reference amounts).

Moreover, the present invention relates to a kit adapted for identifying a subject being susceptible to prostate cancer therapy comprising:
a) a detection agent for at least one miRNA selected from the group consisting of miRNA-375, miRNA-141, miRNA-200b, miRNA-516a-3p and miRNA9*, or of a precursor molecule thereof, and
b) a device adapted for determining the amount of said least one miRNA molecule or of a precursor molecule thereof detected by said detection agent, wherein said device is also adapted for carrying out the comparison of said amount(s) with a reference amount (reference amounts).

The term "kit" as used herein refers to a collection of the aforementioned means, preferably, provided in separately or within a single container. The container, also preferably, comprises instructions for carrying out the method of the present invention. The invention, thus, relates to a kit comprising a means or an agent for measuring a marker referred to herein. Examples for such means or agents as well as methods for their use have been given in this specification. The kit, preferably, contains the aforementioned means or agents in a ready-to-use manner. Preferably, the kit may additionally comprise instructions, e.g., a user's manual for interpreting the results of any determination(s) with respect to the diagnoses provided by the methods of the present invention. Particularly, such manual may include information for allocating the amounts of the determined markers to the kind of diagnosis. Details are to be found elsewhere in this specification. Additionally, such user's s manual may provide instructions about correctly using the components of the kit for determining the amount(s) of the respective biomarker. A user's manual may be provided in paper or electronic form, e.g., stored on CD or CD ROM. The present invention also relates to the use of said kit in any of the methods according to the present invention.

Finally, the present invention relates to the use of at least one miRNA selected from the group consisting of miRNA-375, miRNA-141, miRNA-200b, miRNA-516a-3p and miRNA9*, or of a precursor molecule thereof in a sample from a subject suffering from prostate cancer for staging prostate cancer.

Also envisaged by the present invention is the use of at least one miRNA selected from the group consisting of miRNA-375, miRNA-141, miRNA-200b, miRNA-516a-3p and miRNA9*, or of a precursor molecule thereof in a sample from a subject suffering from prostate cancer for identifying a subject being susceptible to prostate cancer therapy.

Finally, the present invention relates to the use of at least one miRNA selected from the group consisting of miRNA-375, miRNA-141, miRNA-200b, miRNA-516a-3p and miRNA9*, or of a precursor molecule thereof in a blood, blood serum or blood plasma sample from a subject suspected to suffer from prostate cancer for diagnosing prostate cancer.

All references referred to above are herewith incorporated by reference with respect to their entire disclosure content as well as their specific disclosure content explicitly referred to in the above description.

The Figures show:
**Fig. 1**: Circulating miRNA levels in sera from prostate cancer patients. MA-plot of 368 circulating miRNAs. Red, 69 miRNA identified with Limma analysis (p < 0.05, no multiple testing).
**Fig. 2**: Ct values for circulating *miRNA-375* in serum samples taken from patients with metastatic PCa (n=7) and primary PCa (n=13). fdr = 0.036 (test for differential expression; after multiple testing adjustment).
**Fig. 3**: Clustering of 45 serum samples based on the expression levels of 5 circulating miRNAs. Expression levels are color coded in a heatmap. Gleason scores: black, ≥ 8 or M+; dark gray, Gleason 7; light gray, Gleason 6; NM (lymph-node status or metastases): black, N1 or M+; light gray, N0; white, NX; M (metastases): black, M+; light gray, MX.
**Fig. 4****:** Prospective validation of miRNA-375 in 45 samples. miRNA-375 Ct values in serum samples are grouped by a) risk [9 high risk tumors (Gleason ≥ 8 or M+), 21 intermediate risk tumors (Gleason 7) and 15 low risk tumors (Gleason 6)]; and b) lymph-node status (12 tumors N1 or M+ vs. 21 tumors N0). Statistically significant differences were determined using the Wilcoxon test.
**Fig. 5****:** Validation of circulating miRNAs. miRNA Ct values in serum samples grouped by a) risk groups [9 high risk tumors (Gleason ≥ 8 or M+), 21 intermediate risk tumors (Gleason 7) and 15 low risk tumors (Gleason 6)].

The invention will now be illustrated by the following Examples which are not intended to restrict or limit the scope of this invention.

### Example 1:

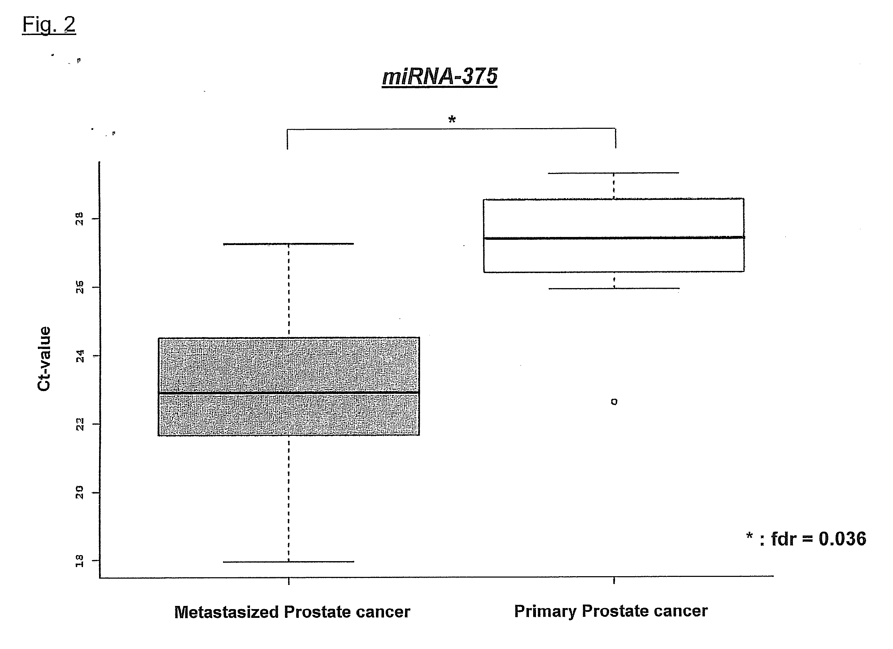

In order to screen for circulating miRNAs associated with prostate cancer progression, 21 1 serum samples were analyzed. RNA was purified, and 667 miRNAs were measured with low density Taq-Man arrays. One sample (IGPS-20) was discarded as an outlier due to the large number of undetermined values (610 out of 768). The overall abundances of circulating miRNAs revealed a significantly higher level of cell-free miRNAs in serum samples taken from patients with metastasized tumors when compared to those with primary prostate cancer (p=0.02, Wilcoxon test). Limma analysis identified 69 miRNAs with higher abundance in this group (p<0.05, without adjusting for multiple testing; Fig. 1). *miRNA-375* was the top marker and the only circulating miRNA which was significantly higher abundant in the sera of patients with distant metastases (fdr = 0.036; Fig 2). The log fold-change between sera of patients with metastatic prostate cancer and patients with primary cancers was considerably high (~ 4 Ct values). All samples were within the measurable range (Ct < 40; Fig 1 and 2).

### Example 2:

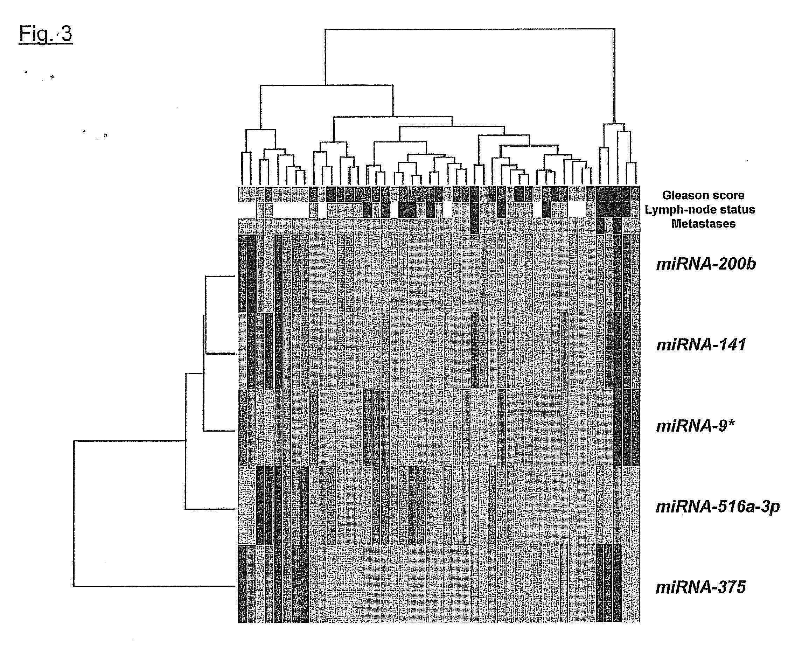

*miRNA-375* and four additional circulating miRNAs (*miRNA-9*, miRNA-141, miRNA-200b and miRNA-516a-3p)* were chosen from the screening study for validation in 45 serum samples of an independent patient cohort. The hierarchical clustering analysis demonstrated that five patients exhibited a high level of selected circulating miRNAs (Fig. 3). The tumors of these patients were characterized by at least one adverse risk factor (high Gleason score, lymph-node involvement or distant metastasis). In contrast, patients with a low risk profile (low grade tumors, organ confined tumors) showed low levels of the five selected miRNAs. *miRNA-141* and *miRNA-200b* were highly correlated (Pearson; r = 0.8; 95%CI = 0.66) in the sample set.

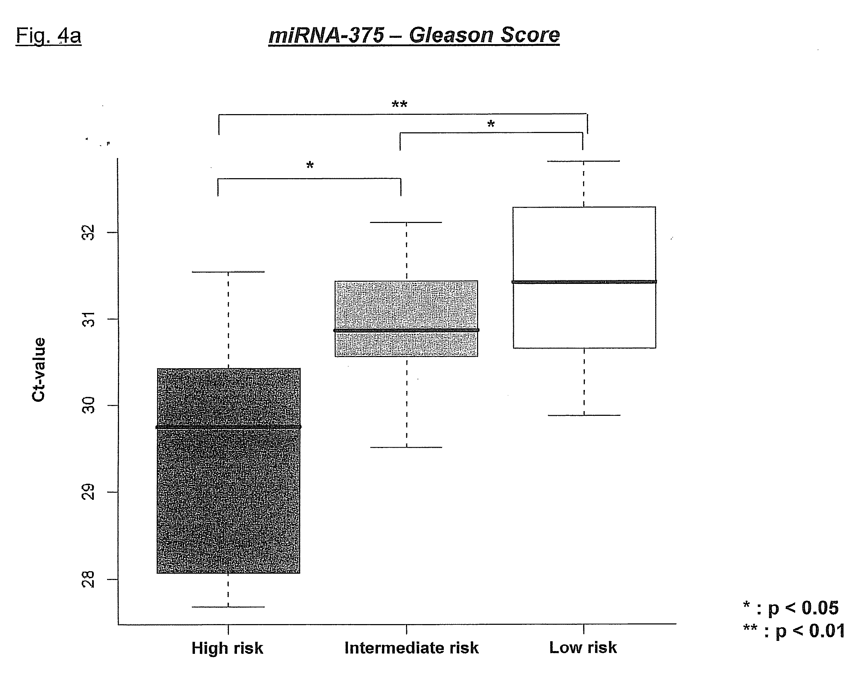

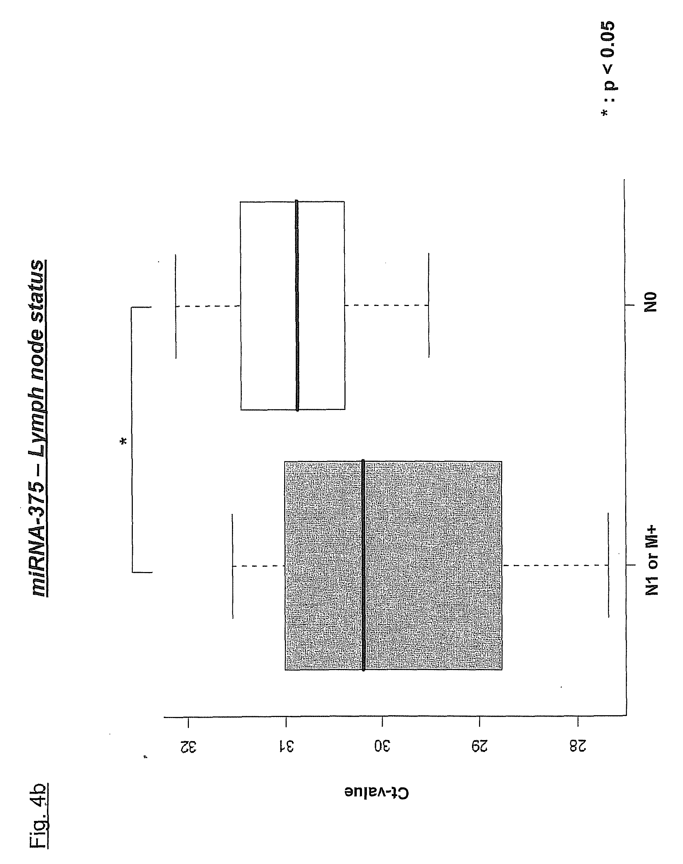

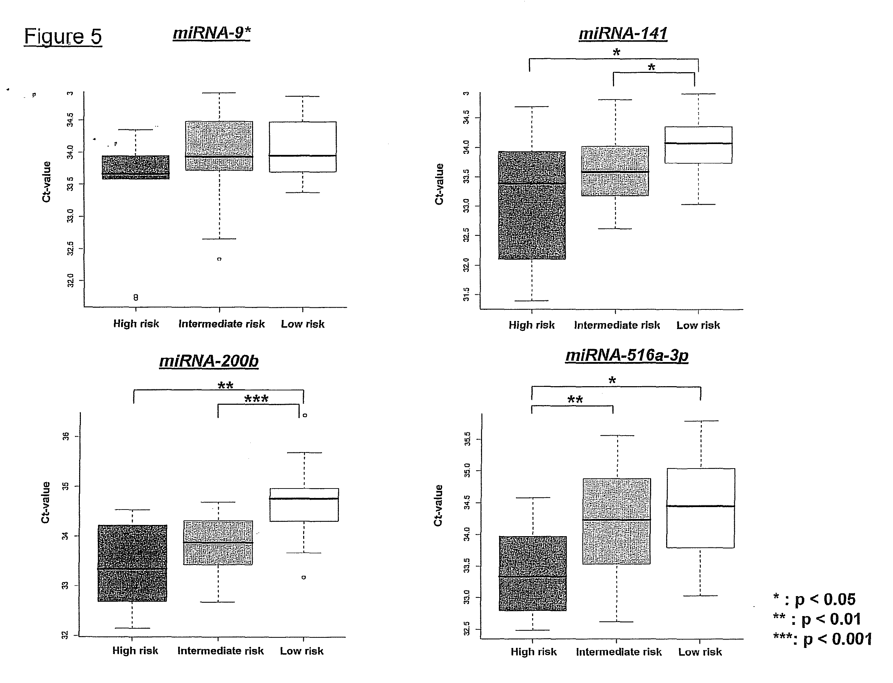

For the analysis, all serum samples were grouped according to the Gleason scores, lymph-node and pT status of the patients. Serum samples from patients with distant metastases were included in the group of Gleason score ≥ 8 tumors and lymph-node positive patients. Different levels of circulating miRNAs were detected in patients whose tumors were Gleason score 6 (low risk), 7 (intermediate risk) and ≥ 8 (high risk; Fig. 5). However, only *miRNA-375* revealed a significant difference in all three groupwise comparisons (Fig. 4a). Additionally, *miRNA-375* was significantly higher abundant in the sera of patients with lymph-node positive status (p = 0.034; Fig 4b) or pathological stage pT3 (p=0.021). The three patients with bone metastases were among those with the four highest abundance values of *miRNA-375* in the serum.

Circulating *miRNA-375* was analyzed in combination with PSA in the validation study. *miRNA-375* levels were considerably higher in the sera of patients with high risk tumors (Gleason Score ≥ 8 or patients with a metastasized prostate cancer) (Fig. 4a). In contrast to that, PSA levels did not distinguish between Gleason 7 and Gleason ≥ 8 tumors or between different lymph-node status. However, the PSA levels were considerably lower in patients with low risk tumors (Gleason score 3+3) when compared to those with Gleason score 7 tumors.

In order to assess the influence of both (*miRNA-375* and PSA) markers on the prediction of clinico-pathological endpoints, an ANOVA analysis was employed. PSA significantly discriminated Gleason 6 and Gleason 7 tumors (Table 1), and the addition of *miRNA-375* further significantly improved the prediction accuracy. In contrast to that, the PSA-based comparison between Gleason 7 and Gleason ≥ 8 tumors showed no significant differences in the ANOVA analysis. However, the introduction of *miRNA-375* into the model lead to an improvement of the predictive power. Additionally, *miRNA-375* improved the prediction of lymph-node and pathological status when added to the PSA prediction model (Table 1).

**Table 1: ANOVA analysis p-Values that the parameters contribute with a significant effect in the ANOVA model. First row: Effect of PSA alone on the discrimination of different pathological parameters. Second row: Additional effect of miRNA-375 to the model already containing PSA.**

| **Marker** | **Gleason 6 vs. 7** | **Gleason 7 vs. ≥8 or M+** | **N1 / M+ vs. N0** | **pT2 vs. pT3** |
|---|---|---|---|---|
| PSA | 0.0053 ** | 0.7967 | 0.1043 | 0.0536. |
| + miRNA-375 | 0.0158 * | 0.0028 ** | 0.0079 ** | 0.0323 * |

## Claims

1. A method for staging prostate cancer in a subj ect suffering from prostate cancer, comprising
a) determining the amount of at least one miRNA selected from the group consisting of miRNA-375, miRNA-141, miRNA-200b, miRNA-516a-3p and miRNA9*, or the amount of a precursor molecule of said at least one miRNA in a sample from said subject,
b) comparing the amount of said at least one miRNA or of said precursor molecule as determined in step a) with a reference amount (with reference amounts), and
c) staging prostate cancer in said subject based on the information obtained in step b).

2. The method of claim 1, wherein said sample is a blood, blood plasma or blood serum sample.

3. The method of claims 1 and 2, wherein said amount is determined by quantitative real-time PCR

4. The method of any one of claims 1 to 3, wherein said staging comprises differentiating between primary prostate cancer and metastasized prostate cancer.

5. The method of any one of claims 1 to 4, wherein an amount of said at least one miRNA or of a precursor molecule thereof larger than the reference amount indicates that said subject suffers from metastasized prostate cancer, and wherein an amount of said at least one miRNA or of a precursor molecule thereof lower than the reference amount indicates that said subject suffers from primary prostate cancer.

6. The method of any one of claims 1 to 3, wherein said staging comprises differentiating between a high risk, an intermediate risk and a low risk.

7. A method for diagnosing the progression of prostate cancer comprising:
(a) determining the amount of least one miRNA selected from the group consisting of miRNA-375, miRNA-141, miRNA-200b, miRNA-516a-3p and miRNA9*, or the amount of a precursor molecule of said at least one miRNA in a first sample from a subject suffering from prostate carcinoma, and
(b) determining the amount of least one miRNA or of said precursor molecule of said at least one miRNA in a second sample of said subject, and
(c) comparing the amount in said first sample with said amount in said second sample.

8. A method for identifying a subject susceptible to prostate cancer therapy, said method comprising
a) determining the amount of at least one miRNA selected from the group consisting of miRNA-375, miRNA-141, miRNA-200b, miRNA-516a-3p and miRNA9*, or the amount of a precursor molecule of said at least one miRNA in a sample from a subject suffering from prostate cancer,
b) comparing the amount of said at least one miRNA or of said precursor molecule as determined in step a) with a reference amount (with reference amounts), and
c) identifying a subject susceptible to prostate cancer therapy.

9. A method for diagnosing prostate cancer in a subject suspected to suffer from prostate cancer, said method comprising
a) determining the amount of at least one miRNA selected from the group consisting of miRNA-375, miRNA-141, miRNA-200b, miRNA-516a-3p and miRNA9*, or the amount of a precursor molecule of said at least one miRNA in a sample from said subject,
b) comparing the amount of said at least one miRNA or of said precursor molecule as determined in step a) with a reference amount (with reference amounts), and
c) diagnosing prostate cancer in said subject based on the information obtained in step b).

10. A device adapted for staging prostate cancer in a subject suffering from prostate cancer comprising:
a) an analysing unit comprising a detection agent for at least one miRNA molecule or of a precursor molecule thereof as defined in claim 1, wherein said analyzing unit is adapted for determining the amount(s) of at said at least one miRNA molecule or of a precursor molecule thereof in a sample detected by the detection agent, and
b) a evaluation unit comprising a computer comprising tangibly embedded a computer program code for carrying out a comparison of the determined amounts obtained from the analyzing unit.

11. A kit adapted for staging prostate cancer in a subject suffering from prostate cancer comprising comprising:
a) a detection agent for at least one miRNA molecule or of a precursor molecule thereof as defined in claim 1, and
b) a device adapted for determining the amount of said least one miRNA molecule or of a precursor molecule thereof detected by said detection agent, wherein said device is also adapted for carrying out the comparison of said amounts as defined in any one of claims 1 to 6.

12. Use of at least one miRNA selected from the group consisting of miRNA-375, miRNA-141, miRNA-200b, miRNA-516a-3p and miRNA9*, or of a precursor molecule thereof in a sample from a subject suffering from prostate cancer for staging prostate cancer.

13. Use of at least one miRNA selected from the group consisting of miRNA-375, miRNA-141, miRNA-200b, miRNA-516a-3p and miRNA9*, or of a precursor molecule thereof in a sample from a subject suffering from prostate cancer for identifying a subject being susceptible to prostate cancer therapy.

14. Use of at least one miRNA selected from the group consisting of miRNA-375, miRNA-141, miRNA-200b, miRNA-516a-3p and miRNA9*, or of a precursor molecule thereof in a sample from a subject suspected to suffer from prostate cancer for diagnosing prostate cancer.
